# EUROPÄISCHE PATENTANMELDUNG

(11) **EP 2 762 622 A2**
(43) Veröffentlichungstag der Anmeldung: **06.08.2014**
(21) Anmeldenummer: 14153273.9
(22) Anmeldetag: 30.01.2014
(51) Int. Cl.: D04C 1/02, A61F 2/04, A61F 2/90

(54) **Gittergeflecht für ein medizinisches Implantat oder Instrument sowie Implantat oder Instrument mit einem derartigen Gittergeflecht**

(30) Priorität: 31.01.2013 DE 102013100984
(71) Anmelder: Acandis GmbH & Co. KG, 76327 Pfinztal (DE)
(72) Erfinder: Mailänder, Werner, 75331 Engelsbrand (DE)
(74) Vertreter: Kilchert, Jochen

(57) **Zusammenfassung**

Die Erfindung betrifft ein Gittergeflecht (10) für ein medizinisches Implantat oder Instrument mit wenigstens zwei Drahtsträngen (11, 12), die miteinander verflochten oder verwebt sind, wobei zumindest ein markierender Drahtstrang (11) wenigstens zwei Verbundmaterialdrähte (13) umfasst, die jeweils einen Kerndraht (14) aus einem röntgensichtbaren Material und eine Ummantelung (15) aus einem Formgedächtnismaterial aufweisen, und wobei die Verbundmaterialdrähte (13) zumindest abschnittsweise parallel zueinander angeordnet sind. Die Erfindung betrifft ferner ein Implantat oder Instrument mit einem derartigen Gittergeflecht.

## Beschreibung

Die Erfindung betrifft ein Gittergeflecht für ein medizinisches Implantat oder Instrument sowie ein Instrument bzw. Implantat mit einem derartigen Gittergeflecht.

Gittergeflechte für medizinische Anwendungen kommen beispielsweise bei der Herstellung von Stents oder Thrombectomiedevices zum Einsatz. Dabei ist es oft erwünscht, dass die Gittergeflechte Einrichtungen aufweisen, die eine verbesserte Röntgensichtbarkeit ermöglichen. Dies ist für die minimalinvasiven Eingriffe relevant, bei welchen die Positionierung der Implantate bzw. Instrumente unter angiografischer Kontrolle stattfinden.

Aus der Praxis bekannt sind Markerelemente in Form von Hülsen, die auf die Drähte bzw. Drahtstränge des Gittergeflechts aufgebracht sind. Diese Hülsen sind meist aus einem Material gebildet, das eine vergleichsweise hohe Röntgensichtbarkeit aufweist. Der Einsatz derartiger röntgensichtbarer Hülsen ist jedoch mit dem Nachteil verbunden, dass die Hülsen zusätzlich auf die Wandstärke des Implantats auftragen, die Wandstärke also bereichsweise erhöhen. Überdies unterliegen Materialien, die für die röntgensichtbaren Markerelemente eingesetzt werden, einer erhöhten Korrosion, wenn sie mit Körperflüssigkeiten in Kontakt gelangen.

Ein weiterer Nachteil der bekannten Markerelemente besteht darin, dass diese lediglich eine punktuelle Erhöhung der Röntgensichtbarkeit bereitstellen. Somit ist es oft schwierig, den Verlauf eines Implantats in einem Körperhohlorgan, beispielsweise einem Blutgefäß, zu erkennen.

Die Aufgabe der Erfindung besteht darin, ein Gittergeflecht für medizinische Implantate oder Instrumente anzugeben, das eine gute Röntgensichtbarkeit über einen längeren Abschnitt des Gittergeflechts aufweist. Ferner besteht die Aufgabe der Erfindung darin, ein Implantat oder Instrument mit einem derartigen Gitter-geflecht anzugeben.

Erfindungsgemäß wird diese Aufgabe im Hinblick auf das Gittergeflecht durch den Gegenstand des Patentanspruchs 1 und im Hinblick auf das Implantat oder Instrument durch den Gegenstand des Patentanspruchs 9 gelöst.

Die Erfindung beruht auf dem Gedanken, ein Gittergeflecht für ein medizinisches Implantat oder Instrument mit wenigstens zwei Drahtsträngen anzugeben, die miteinander verflochten oder verwebt sind. Zumindest ein markierender Drahtstrang umfasst wenigstens zwei Verbundmaterialdrähte, die jeweils einen Kerndraht aus einem röntgensichtbaren Material und eine Ummantelung aus einem Formgedächtnismaterial aufweisen. Die Verbundmaterialdrähte sind zumindest abschnittsweise parallel zueinander angeordnet.

Es hat sich gezeigt, dass Drähte aus einem Verbundmaterial, die im Rahmen der Anmeldung als Verbundmaterialdrähte bezeichnet werden, einerseits die Anforderungen an die Korrosionsbeständigkeit erfüllen, da die Ummantelung das röntgensichtbare Material von dem Einfluss von Körperflüssigkeiten abschirmt. Andererseits ermöglichen die Verbundmaterialdrähte mit einem Kerndraht aus einem röntgensichtbaren Material die Darstellung des Gittergeflechts unter Röntgenkontrolle. Dabei ist es besonders vorteilhaft, dass die Drähte Teil eines der Drahtstränge sind und sich somit zumindest abschnittsweise entlang des Gittergeflechts erstrecken. Unter Röntgenkontrolle ist somit auch der Verlauf des Gittergeflechts erkennbar.

Die Verwendung von wenigstens zwei Verbundmaterialdrähten ist zweckmäßig, um eine ausreichende Darstellung unter Röntgenkontrolle zu ermöglichen. Insbesondere trägt die Verwendung von wenigstens zwei Verbundmaterialdrähten dem Umstand Rechnung, dass moderne, digitale Angiografiegeräte eine begrenzte Auflösung aufweisen. Die Bilderfassung erfolgt über ein Pixelarray, wobei die einzelnen Pixel durch das Auftreffen von Röntgenstrahlen aktiviert werden. Röntgensichtbare Materialien schirmen die Röntgenstrahlen ab, so dass die von derartigen Materialien verdeckten Pixel nicht aktiviert werden. Die nicht aktivierten Pixel repräsentieren somit in der nachfolgenden Bilddarstellung den Verlauf des röntgensichtbaren Materials. Wegen den kleinen Kerndrahtdurchmessern der Verbundmaterialdrähte werden die Pixel des Angiografiegeräts oft nicht vollständig von Röntgenstrahlen abgeschirmt, so dass die Gefahr besteht, dass einzelne Pixel trotz Überdeckung durch einen Verbundmaterialdraht aktiviert werden. Durch die Verwendung von zwei parallel zueinander angeordneten Verbundmaterialdrähten wird dies vermieden. Die parallele Anordnung von zwei Verbundmaterialdrähten erhöht somit die Röntgensichtbarkeit des Gittergeflechts.

In diesem Zusammenhang wird darauf hingewiesen, dass der Kerndraht und die Ummantelung der Verbundmaterialdrähte fest und vollflächig miteinander verbunden sind. Insbesondere können die Materialien des Kerndrahts und der Ummantelung stoffschlüssig miteinander verbunden sein.

Im Allgemeinen kann vorgesehen sein, dass das Gittergeflecht wenigstens 24, insbesondere wenigstens 36, insbesondere wenigstens 48, insbesondere wenigstens 62, Drahtstränge aufweist oder aus einer derartigen Anzahl von Drahtsträngen gebildet ist. Durch die Anzahl der eingesetzten Drahtstränge kann die Feinmaschigkeit des Gittergeflechts eingestellt werden. Vorzugsweise sind wenigstens 2, insbesondere wenigstens 4, insbesondere wenigstens 6, insbesondere wenigstens 8, insbesondere wenigstens 12, der Drahtstränge als markierende Drahtstränge ausgebildet, die insbesondere jeweils wenigstens zwei Verbundmaterialdrähte umfassen.

Grundsätzlich kann ein markierender Drahtstrang bzw. jeder markierende Drahtstrang mehr als zwei Verbundmaterialdrähte aufweisen bzw. daraus gebildet sein. Beispielsweise kann ein markierender Drahtstrang bzw. jeder markierende Drahtstrang, wenigstens drei, insbesondere wenigstens vier, Verbundmaterialdrähte umfassen oder aus einer derartigen Anzahl von Verbundmaterialdrähten gebildet sein.

Bei einer bevorzugten Ausführungsform der Erfindung sind die Verbundmaterialdrähte zueinander parallel in einer Geflechtebene des Gittergeflechts angeordnet. Damit ist sichergestellt, dass die Verbundmaterialdrähte nicht zusätzlich auf die Wandstärke des Gittergeflechts Einfluss nehmen. Die parallele Anordnung in der Geflechtebene des Gittergeflechts ermöglicht vielmehr die Einbettung von röntgensichtbaren Materialien in das Gittergeflecht ohne eine Erhöhung der Wandstärke des Gittergeflechts.

Je nach gewünschtem Anwendungsfall weist das Gittergeflecht vorzugsweise Drahtstränge mit einer unterschiedlichen Anzahl von Drähten und einem unterschiedlichen Querschnittsdurchmesser der einzelnen Drähte auf. Der Fachmann wählt die Drahtdurchmesser insbesondere in Abhängigkeit der gewünschten Wandstärke, Geflechtdichte und/oder Radialkraft. In konkreten Anwendungsfällen ist es vorteilhaft, wenn die Verbundmaterialdrähte jeweils einen Querschnittsdurchmesser zwischen 10 µm und 70 µm, insbesondere zwischen 15 µm und 60 µm, insbesondere zwischen 30 µm und 50 µm, insbesondere zwischen 40 µm und 45 µm, vorzugsweise 43 µm, aufweisen.

Der Querschnittsdurchmesser des Kerndrahts kann in bevorzugten Ausführungsformen und in Abhängigkeit des jeweiligen Anwendungsfalls zwischen 5 µm und 50µm, insbesondere zwischen 10 µm und 40 µm, insbesondere zwischen 15 µm und 30 µm, insbesondere zwischen 20 µm und 25 µm, vorzugsweise 23 µm, aufweisen.

Generell ist es zweckmäßig, wenn sich die Verbundmaterialdrähte zumindest abschnittsweise, insbesondere vollständig, entlang ihrer parallelen Anordnung berühren. Damit ist sichergestellt, dass der Abstand zwischen den Kerndrähten ausreichend klein ist, um die Aktivierung eines einzelnen Pixels des Angiografiegeräts zu vermeiden. Mit Blick auf moderne Angiografiegeräte hat es sich als vorteilhaft herausgestellt, wenn der Abstand zwischen den Kerndrähten zweier benachbarter Verbundmaterialdrähte höchstens 40 µm, insbesondere höchstens 30 µm, insbesondere höchstens 25 µm, insbesondere höchstens 20 µm, insbesondere höchstens 15 µm, beträgt.

Als röntgensichtbares Material für den Kerndraht sind im Rahmen der Anmeldung insbesondere Gold, Silber, Platin und/oder Tantal vorgesehen. Mit anderen Worten weist der Kerndraht vorzugsweise Gold und/oder Silber und/oder Platin und/oder Tantal auf oder besteht daraus.

Die Ummantelung weist hingegen ein Formgedächtnismaterial auf, das insbesondere eine Nickeltitanlegierung, vorzugsweise Nitinol, sein kann. Die Ummantelung kann auch aus einer Nickeltitanlegierung, insbesondere Nitinol, bestehen.

Gemäß einem nebengeordneten Aspekt beruht die Erfindung auf dem Gedanken, ein medizinisches Implantat, insbesondere einen Stent, oder ein medizinisches Instrument, insbesondere ein Thrombectomiedevice, mit einem zuvor erläuterten Gittergeflecht anzugeben. Die im Zusammenhang mit dem Gittergeflecht genannten vorteilhaften Weiterbildungen und Vorteile gelten entsprechend für das medizinische Implantat bzw. das medizinische Instrument. Besonders hervorzuheben ist der Vorteil, dass bei einem Stent oder Thrombectomiedevice ein Gittergeflecht mit wenigstens einem markierenden Drahtstrang, der aus zwei oder mehr Verbundmaterialdrähten gebildet ist, dessen Verlauf in einem Blutgefäß unter Röntgenkontrolle gut erkennbar ist.

In einer bevorzugten Weiterbildung des erfindungsgemäßen Implantats oder Instruments bildet das Gittergeflecht eine Geflechtebene, wobei die Verbundmaterialdrähte des markierenden Drahtstrangs parallel zueinander in der Geflechtebene angeordnet sind. Die Geflechtebene entspricht insbesondere der Wandungsebene des Implantats oder Instruments. Dies gilt insbesondere für die Wandungsebene eines Stents oder Thrombectomiedevices. Bei derartigen Implantaten oder Instrumenten erstreckt sich das Gittergeflecht meist zylinderartig entlang einer Längsachse. Die Wandungsebene bzw. Geflechtebene des Gittergeflechts bildet also einen Kreiszylinder.

Ein weiterer nebengeordneter Aspekt der Erfindung betrifft eine Set mit einem zuvor beschriebenen Implantat oder Instrument. Das Set umfasst zusätzlich eine Schleuse, in welcher das Implantat oder das Instrument längsverschieblich gelagert bzw. angeordnet ist. Die Schleuse kann mit einem Katheter verbindbar sein, um das Implantat oder Instrument zum Zwecke der Zuführung in ein Körperhohlorgan, beispielsweise ein Blutgefäß, in den Katheter einzuführen. Das Implantat ist innerhalb des Sets mit einem Transportdraht lösbar verbunden, insbesondere derart, dass das Implantat am Behandlungsort vom Transportdraht abkoppelbar ist. Das Instrument ist hingegen fest mit einem Betätigungselement, insbesondere einem Führungsdraht, verbunden, so dass das Instrument mit Hilfe des Betätigungselements über einen Katheter zurückziehbar ist.

Die Erfindung wird im Folgenden unter Bezugnahme auf die beigefügten, schematischen Zeichnungen näher erläutert. Darin zeigen
- Figur 1:: einen Ausschnitt eines erfindungsgemäßen Gittergeflechts mit einem markierenden Drahtstrang;
- Figur 2:: eine Querschnittsansicht eines einzelnen Verbundmaterialdrahts unter Röntgenkontrolle;
- Figur 3:: eine Querschnittsansicht eines markierenden Drahtstrangs mit zwei Verbundmaterialdrähten unter Röntgenkontrolle; und
- Figur 4:: eine Querschnittsansicht eines Verbundmaterialdrahts des Gittergeflechts gemäß Figur 1.

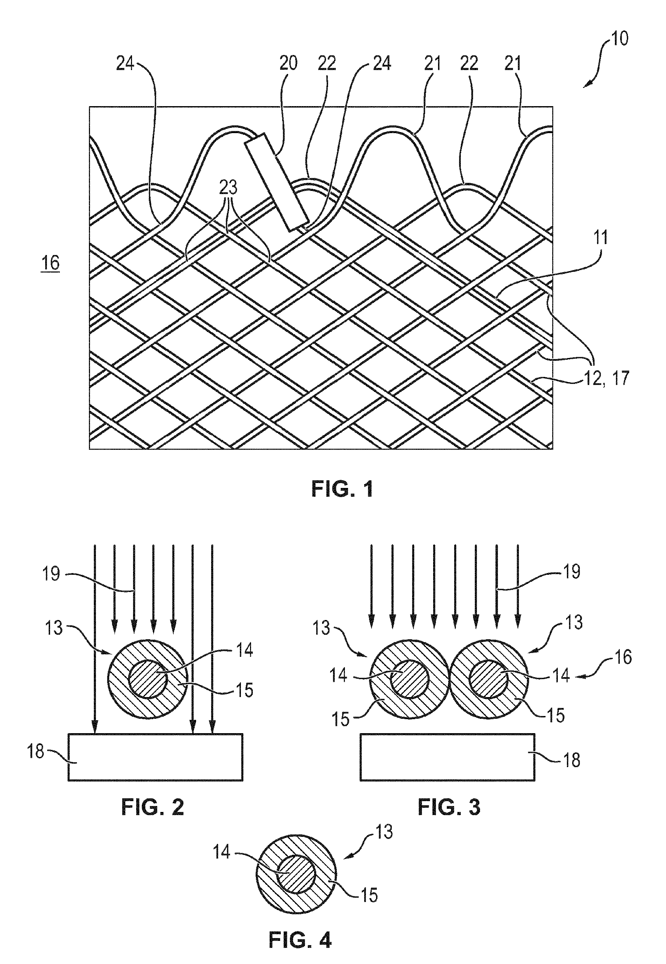

In Fig. 1 ist ein Ausschnitt eines Gittergeflechts 10 dargestellt. Das Gittergeflecht 10 ist aus mehreren Drahtsträngen 11, 12 gebildet, wobei sich die Drahtstränge an Kreuzungspunkten 23 über- und unterkreuzen. Mit anderen Worten sind die Drahtstränge 11, 12 miteinander verflochten bzw. verwebt.

Grundsätzlich können die einzelnen Drahtstränge 11, 12 in unterschiedlichen Konfigurationen miteinander verflochten sein. Beispielsweise kann eine 1-über-1-Flechtung vorgesehen sein. Das bedeutet, dass entlang eines Drahtstrangs 11, 12 jeweils ein erster kreuzender Drahtstrang über und ein zweiter, auf den ersten Drahtstrang 11, 12 unmittelbar folgender Drahtstrang 11, 12, unterkreuzt wird. Andere Flechtarten sind möglich. So zeigt Fig. 1 beispielsweise eine 2-über-2-Flechtung, bei der jeweils zwei parallel zueinander und unmittelbar benachbarte Drahtstränge 11, 12 überkreuzt und die nachfolgenden, parallel zueinander angeordneten und benachbarten Drahtstränge 11, 12 unterkreuzt werden. Mit anderen Worten verläuft ein Drahtstrang 11, 12 jeweils über zwei kreuzende Drahtstränge 11, 12, um im weiteren Verlauf zwei weitere Drahtstränge 11, 12 zu unterkreuzen.

Besonders bevorzugt ist bei allen Ausführungsformen der Erfindung eine 1-über-2-Flechtung. Dabei über- und unterkreuzt jeweils ein Drahtstrang 11, 12 zwei kreuzende Drahtstränge 11, 12. Das hat den Vorteil, dass der einzelne, jeweils zwei Drahtstränge 11, 12 oberseitig oder unterseitig überkreuzende Drahtstrang 11, 12 eine vergleichsweise geringe Verformung erfährt. Dieser Vorteil kommt insbesondere bei Ausführungsformen zur Geltung, bei welchen der einzelne über oder unter zwei kreuzenden Drahtstränge 11, 12 verlaufende Drahtstrang 11, 12 ein markierender Drahtstrang 11 ist. Die Verbundmaterialdrähte 13 des markierenen Drahtstrangs 11 werden somit geschont, insbesondere nicht wesentlich verformt.

Die Drahtstränge 11, 12 können jeweils eine unterschiedliche Anzahl von Drähten aufweisen. Jeder Drahtstrang 11, 12 weist wenigstens einen Draht auf. Dabei ist insbesondere vorgesehen, dass ein Großteil der Drähte des Gittergeflechts 10 als Einzeldrähte 17 ausgebildet ist. Im Rahmen der Anmeldung werden als Einzeldrähte 17 Drähte bezeichnet, die aus einem einzigen Material gebildet sind. Im Unterschied dazu umfasst das Gittergeflecht 10 auch Drahtstränge 11, 12 mit Verbundmaterialdrähten 13. Verbundmaterialdrähte 13 umfassen wenigstens zwei unterschiedliche Materialien. Konkret ist vorgesehen, dass ein Verbundmaterialdraht 13 einen Kerndraht 14 und eine Ummantelung 15 aufweist. Der Kerndraht 14 ist vorzugsweise aus einem röntgensichtbaren Material gebildet. Die Ummantelung 15 umfasst hingegen ein Formgedächtnismaterial bzw. allgemein ein biokompatibles, insbesondere korrosionsbeständiges, Material.

Wie in Fig. 1 gut erkennbar ist, ist das Gittergeflecht 10 aus einer Vielzahl von Drahtsträngen 11, 12 gebildet, wobei der Großteil der Drahtstränge 11, 12 nur eine stützende Funktion aufweist. Diese Drahtstränge 11, 12 werden im Rahmen der Anmeldung als stützende Drahtstränge 12 bezeichnet. Die stützenden Drahtstränge 12 können ein oder mehrere Einzeldrähte 17 aufweisen. Bei dem dargestellten Ausführungsbeispiel weisen die stützenden Drahtstränge 12 jeweils einen einzigen Einzeldraht 17 auf. Die stützenden Drahtstränge 12 sind miteinander verflochten.

Ferner ist ein markierender Drahtstrang 11 vorgesehen, der in das Gittergeflecht 10 eingeflochten ist. Der markierende Drahtstrang 11 kann sowohl zusätzlich zu den stützenden Drahtsträngen 12 eingeflochten sein oder, wie in Fig. 1 dargestellt ist, einen stützenden Drahtstrang 12 ersetzen. Der markierende Drahtstrang 11 umfasst wenigstens zwei Verbundmaterialdrähte 13. Die Verbundmaterialdrähte 13 des markierenden Drahtstrangs 11 sind parallel zueinander angeordnet. bzw. verlaufen parallel zueinander. Dabei berühren sich die Verbundmaterialdrähte 13 über ihre gesamte Länge. Mit anderen Worten ist der markierende Drahtstrang 11 aus einem Verbundmaterialdrahtpaar gebildet.

Vorzugsweise erstreckt sich der markierende Drahtstrang 11 über die gesamte Länge des Gittergeflechts 10, also von einem ersten Längsende bis zu einem zweiten Längsende. An den Längsenden ist der markierende Drahtstrang 11, wie in Fig. 1 gut erkennbar ist, umgelenkt und in das Geflecht zurückgeführt. Auf diese Weise ist eine Endschlaufe 21, 22 gebildet.

Das Gittergeflecht gemäß Fig. 1 umfasst unterschiedliche Endschlaufen 21, 22 am Längsende. Generell sind alle Drahtstränge 11, 12 an wenigstens einem Längsende des Gittergeflechts 10 umgelenkt und bilden eine Endschlaufe 21, 22. Jede zweite Endschlaufe 21, 22 ist als geflechtbegrenzende Endschlaufe 22 gebildet. Jeweils zwischen zwei geflechtsbegrenzenden Endschlaufen 22 ist eine vorstehende Endschlaufe 21 vorgesehen. Die vorstehenden Endschlaufen 21 sind aus stützenden Drahtsträngen 12 gebildet. Zur Bildung der vorstehenden Endschlaufe 21 ist der Einzeldraht 17 des stützenden Drahtstrangs 12 auf Höhe der ersten Kreuzungspunkte 23 der Drahtstränge 11, 12 umgelenkt. Mit anderen Worten weist der Einzeldraht 17 des stützenden Drahtstrangs 12 wenigstens eine, insbesondere zwei Umlenkungen 24 auf, wodurch der parallele Verlauf benachbarter Drahtstränge durchbrochen wird. Zwischen den beiden Umlenkungen 24 des Einzeldrahts 17 bzw. des stützenden Drahtstrangs 12 ist die vorstehende Endschlaufe 21 ausgeformt.

Im Bereich der vorstehenden Endschlaufen 21 kann, zumindest an einzelnen vorstehenden Endschlaufen 21, ein zusätzliches röntgensichtbares Markerelement in Form einer Markerhülse 20 angeordnet sein. Dadurch wird sichergestellt, dass das Längsende des Gittergeflechts 10 unter Röntgenkontrolle gut erkennbar ist.

Die Verbundmaterialdrähte 13 sind aus zwei unterschiedlichen, vorzugsweise stoffschlüssig miteinander verbundenen, Schichten bzw. Materialien aufgebaut. Fig. 4 zeigt einen Querschnitt durch einen Verbundmaterialdraht 13. Der Verbundmaterialdraht 13 umfasst einen Kerndraht 14, der ein röntgensichtbares Material, beispielsweise Gold, Silber, Tantal oder Platin, aufweist. Der Kerndraht 14 ist von einer Ummantelung 15 umgeben, die vorzugsweise aus einer Nickeltitanlegierung, insbesondere aus Nitinol, gebildet. Der Kerndraht weist vorzugsweise einen Querschnittsdurchmesser von 23 µm auf. Der Drahtdurchmesser des gesamten Verbundmaterialdrahts 13 beträgt vorzugsweise 43 µm. Folglich ist für die Ummantelung 15 eine Schichtdicke von etwa 20 µm vorgesehen.

Die Figuren 2 und 3 zeigen die Wirkung der parallel zueinander angeordneten Verbundmaterialdrähte 13. Die parallele Anordnung der Verbundmaterialdrähte 13 bezieht sich vorzugsweise auf die Geflechtebene 16. Als Geflechtebene 16 wird im Rahmen der Anmeldung die Ebene bezeichnet, in welcher sich das Geflecht hauptsächlich erstreckt. Die Geflechtebene 16 ist nicht zwingenderweise flach, sondern kann auch gekrümmt sein. In Fällen, bei welchen das Gittergeflecht beispielsweise als Stent eingesetzt wird, ist die Geflechtebene 16 vorzugsweise zylinderförmig gekrümmt. Die Geflechtebene 16 kann sich auch trichterförmig erstrecken, beispielsweise bei Einsatz des Gittergeflechts als Thrombectomiedevice. Insofern ist die Geflechtebene 16 mit der Wandungsebene des entsprechenden medizinischen Implantats oder Instruments gleichzusetzen, das aus einem Gittergeflecht 10 gebildet ist.

In den Figuren 2 und 3 ist jeweils ein Bilderfassungspixel 18 gezeigt. Das Bilderfassungspixel 18 ist durch Einwirkung von Röntgenstrahlen 19 aktivierbar. Im aktivierten Zustand, wie er in Fig. 2 dargestellt ist, wird ein entsprechendes Signal an das Angiografiegerät ausgesandt, so dass in der bildlichen Darstellung ein entsprechend zugeordnetes Bilddarstellungspixel kein röntgensichtbares Material anzeigt. Wird das Bilderfassungspixel 18 hingegen nicht durch Röntgenstrahlen 19 getroffen, wie dies in Fig. 3 gezeigt ist, wird das zugehörige Bilddarstellungspixel auf dem Bildschirm des Angiografiegeräts so konfiguriert, dass für den Benutzer ein Bauteil, konkret der Kerndraht 14, erkennbar ist.

Fig. 2 zeigt die Problematik, wenn der Verbundmaterialdraht 13 als einzelner Draht vorliegt. Wegen des geringen Kerndurchmessers werden nicht alle Röntgenstrahlen 19 vom Bilderfassungspixel 18 abgeschirmt. Es erreichen ausreichend viele Röntgenstrahlen 19 das Bilderfassungspixel 18, so dass das Bilderfassungspixel 18 aktiviert wird. Trotz des Vorhandenseins eines röntgensichtbaren Materials im Röntgenstrahl 19 wird dies am Angiografiegerät also nicht erfasst.

Um dies zu vermeiden, ist bei der Erfindung vorgesehen, zwei Verbundmaterialdrähte 13 in paralleler Anordnung in das Gittergeflecht 10 einzuflechten. Dabei ist es besonders bevorzugt, wenn der Abstand zwischen den Kerndrähten 14 zweier benachbarter Verbundmaterialdrähte 13 möglichst klein ist. Vorzugsweise beträgt der Abstand zwischen den Kerndrähten 14 benachbarter Verbundmaterialdrähte 13 höchstens 20 µm, insbesondere höchstens 18 µm, insbesondere höchstens 15 µm, insbesondere höchstens 10 µm. So ist gewährleistet, dass das Bilderfassungspixel 18 ausreichend von Röntgenstrahlen 19 abgeschirmt ist und richtigerweise das Vorhandensein eines röntgensichtbaren Materials signalisiert. Die Sichtbarkeit der Verbundmaterialdrähte 13 wird also dadurch erhöht, dass sichergestellt wird, dass die für die Erfassung der Verbundmaterialdrähte 13 erforderlichen Bilderfassungspixel 18 effizient von Röntgenstrahlen 19 abgeschirmt werden.

Das Gittergeflecht 10 ist für unterschiedliche Implantate oder Instrumente einsetzbar. Besonders bevorzugt ist der Einsatz des Gittergeflechts 10 als Stent oder Thrombectomiedevice. In beiden Fällen ist das Gittergeflecht 10 vorzugsweise hohlzylindrisch geformt bzw. bildet einen hohlzylindrischen oder rotationssymmetrischen Körper. Alternativ kann das Gittergeflecht 10 auch zur Behandlung von Aneurysmen eingesetzt werden, wobei das Gittergeflecht 10 in einer flachen Konfiguration vorliegen kann.

### Bezugszeichenliste

- 10: Gittergeflecht
- 11: markierender Drahtstrang
- 12: stützender Drahtstrang
- 13: Verbundmaterialdraht
- 14: Kerndraht
- 15: Ummantelung
- 16: Geflechtebene
- 17: Einzeldraht
- 18: Bilderfassungspixel
- 19: Röntgenstrahl
- 20: Markerhülse
- 21: vorstehende Endschlaufe
- 22: geflechtbegrenzende Endschlaufe
- 23: Kreuzungspunkt
- 24: Umlenkung

## Patentansprüche

1. Gittergeflecht (10) für ein medizinisches Implantat oder Instrument mit wenigstens zwei Drahtsträngen (11, 12), die miteinander verflochten oder verwebt sind, wobei zumindest ein markierender Drahtstrang (11) wenigstens zwei Verbundmaterialdrähte (13) umfasst, die jeweils einen Kerndraht (14) aus einem röntgensichtbaren Material und eine Ummantelung (15) aus einem Formgedächtnismaterial aufweisen, und wobei die Verbundmaterialdrähte (13) zumindest abschnittsweise parallel zueinander angeordnet sind.

2. Gittergeflecht (10) nach Anspruch 1,
**dadurch gekennzeichnet, dass**
die Verbundmaterialdrähte (13) zueinander parallel in einer Geflechtebene (16) des Gittergeflechts (10) angeordnet sind.

3. Gittergeflecht (10) nach Anspruch 1 oder 2,
**dadurch gekennzeichnet, dass**
die Verbundmaterialdrähte (13) jeweils einen Querschnittsdurchmesser zwischen 10 µm und 70 µm, insbesondere zwischen 15 µm und 60 µm, insbesondere zwischen 30 µm und 50 µm, insbesondere zwischen 40 µm und 45 µm, vorzugsweise 43 µm, aufweisen.

4. Gittergeflecht (10) nach einem der vorhergehenden Ansprüche,
**dadurch gekennzeichnet, dass**
der Kerndraht (14) einen Querschnittsdurchmesser zwischen 5 µm und 50 µm, insbesondere zwischen 10 µm und 40 µm, insbesondere zwischen 15 µm und 30 µm, insbesondere zwischen 20 µm und 25 µm, vorzugsweise 23 µm, aufweist.

5. Gittergeflecht (10) nach einem der vorhergehenden Ansprüche,
**dadurch gekennzeichnet, dass**
sich die Verbundmaterialdrähte (13) zumindest abschnittsweise, insbesondere vollständig, entlang ihrer parallelen Anordnung berühren.

6. Gittergeflecht (10) nach einem der vorhergehenden Ansprüche,
**dadurch gekennzeichnet, dass**
der Abstand zwischen den Kerndrähten (14) unmittelbar benachbarter Verbundmaterialdrähte (13) höchstens 40 µm, insbesondere höchstens 30 µm, insbesondere höchstens 25 µm, insbesondere höchstens 20 µm, insbesondere höchstens 15 µm, beträgt.

7. Gittergeflecht (10) nach einem der vorhergehenden Ansprüche,
**dadurch gekennzeichnet, dass**
der Kerndraht (14) Gold und/oder Silber und/oder Platin und/oder Tantal aufweist oder daraus besteht.

8. Gittergeflecht (10) nach einem der vorhergehenden Ansprüche,
**dadurch gekennzeichnet, dass**
die Ummantelung (15) eine Nickel-Titan-Legierung, insbesondere Nitinol, aufweist oder daraus besteht.

9. Medizinisches Implantat, insbesondere Stent, oder medizinisches Instrument, insbesondere Thrombektomiedevice, mit einem Gittergeflecht (10) nach einem der vorhergehenden Ansprüche.

10. Implantate oder Instrument nach Anspruch 9,
**dadurch gekennzeichnet, dass**
das Gittergeflecht (10) eine Geflechtebene (16) bildet, wobei die Verbundmaterialdrähte (13) des markierenden Drahtstrangs (11) parallel zueinander in der Geflechtebene (16) angeordnet sind.

11. Set mit einem Implantat nach Anspruch 9 oder 10, wobei das Implantat lösbar mit einem Transportdraht verbunden und längsverschieblich in einer Schleuse angeordnet ist.

12. Set mit einem Instrument nach Anspruch 9 oder 10, wobei das Instrument fest mit einem Betätigungselement, insbesondere einem Führungsdraht, verbunden und längsverschieblich in einer Schleuse angeordnet ist.
